# EUROPEAN PATENT APPLICATION

(11) **EP 4 534 027 A1**
(43) Date of publication of application: **09.04.2025**
(21) Application number: 23811226.2
(22) Date of filing: 22.05.2023
(51) Int. Cl.: A61B 17/42

(54) **VAGINAL PROBE FOR PERFORMING COLPOTOMIES**

(30) Priority: 25.05.2022 ES 202200176 U
(71) Applicant: Ponce Sebastia, Jordi, 08024 Barcelona (ES)
(72) Inventor: PONCE SEBASTIÀ, Jordi, 08024 Barcelona (ES)
(74) Representative: Sahuquillo Huerta, Jesús
(86) International application number: PCT/ES2023/070330
(87) International publication number: WO 2023/227814

(57) **Abstract**

Vaginal probe for colpotomies which, being placed under direct endoscopic vision through it, allows the absence of trauma to the cervix, which it embraces and protects, even in a watertight manner when necessary, such as in cervical cancer surgery, without an uterine stalk and therefore with no possibility of perforation injuries, especially in endometrial cancer operations, and also having good pulsation of the vaginal fornices with the possibility of backlighting and marking of the surface where the vaginal section is to be performed. The probe is made up of at least three distinct parts: a head (1) at the distal end, a central stem (2) and an ergonomic handle (3), which can house a light source, being made in a single piece of a single transparent material to allow light to pass through.

## Description

### TECHNICAL SECTOR

The present invention falls within the field of medicine and surgery, specifically in the field of instruments for gynaecological surgery.

### BACKGROUND TO THE INVENTION

A hysterectomy is the surgical resection of a woman's uterus or womb and may include the complete removal of the body, fundus and cervix, with the cervix remaining in some cases. This type of surgery may be necessary in pathologies such as cervical cancer and cancer of the body of the uterus, as well as other benign pathologies such as hypertrophic uterus, myomas or hyperplasia.

In the practice of gynaecological surgical procedures including hysterectomy, using an endoscopic procedure with minimally invasive surgery, whether laparoscopic or robotic, the placement of a uterine manipulator or mobiliser through the vagina is very common and useful.

The vast majority of manipulators include a stem that is placed inside the uterus and which, among other utilities, serves to guide the correct placement of a cup that must mark the vaginal fornices in the last step of the surgery, called colpotomy, where the circular section of the vagina over this cup allows the complete excision of the uterus, for subsequent extraction through the vagina itself after removal of the manipulator. There are currently tools of this type available from manufacturers such as Clermont-Ferrand^{®} (Storz), Rumi II^{®} or RumiArch^{®} (Cooper Surgical) or V-Care^{®}, among others.

Following the publication of the LACC study (DOI: 10.1056/NEJMoa1806395) the oncological results of minimally invasive surgery in cases of cervical cancer have been called into question. Multiple causative factors that have been postulated include the placement of the manipulator with an endo-uterine stalk and therefore in constant, damaging contact with the tumour, which is also exposed to the pelvic cavity prior to transvaginal removal. By extension, the inappropriateness of the use of these devices has extended beyond cervical cancer to endometrial cancer, which is the most prevalent genital cancer tumour in developed countries, including Spain.

Consequently, it has become necessary to work in these surgical practices avoiding the introduction of any element through the cervix into the uterine cavity.

However, the advantages of having a transvaginal insertion instrument that is precisely located in the vaginal fornices, hugging and protecting the cervix, that presses cranially on the hysterectomy specimen, avoiding the proximity of the ureters and therefore their possible injury, and that at the same time allows visualising and deciding the most appropriate point for the colpotomy or final section of the vagina, are obvious.

The instrument should avoid placing anything inside the cervical canal or the uterine cavity, ensuring correct positioning around the cervix without injuring it.

Furthermore, multiple publications postulate that, in the case of cervical cancer, isolation of the tumoural cervix should be guaranteed, avoiding, in addition to trauma, any exposure of the tumour to the interior of the pelvic cavity, i.e. the cervix should be completely protected and isolated before vaginal section or colpotomy.

Various surgical manoeuvres have been described for this purpose, all of them complex and none of them based on a new device that makes it extremely easy to achieve this goal.

This is why there is an unmet need in gynaecological surgical practice for instruments which, being placed under direct endoscopic vision through it, allow the absence of trauma to the cervix, which it embraces and protects, even in a watertight manner when necessary, such as in cervical cancer surgery, without endo-uterine stalk and therefore with the impossibility of perforation injuries, especially in endometrial cancer surgery, and also having a good pulsation of the vaginal fornices with the possibility of backlighting and marking the surface where the vaginal section is to be performed.

### EXPLANATION OF THE INVENTION

The object of the present invention is to obtain a vaginal probe for performing colpotomies which, being placed under direct endoscopic vision through it, allows the absence of trauma to the cervix, which it embraces and protects, even in a watertight manner when necessary, such as in cervical cancer surgery, without endo-uterine stalk and therefore with the impossibility of perforation injuries, especially in endometrial cancer operations, and also having a good pulsation of the vaginal fornices with the possibility of backlighting and marking the surface where the vaginal section is to be performed.

The vaginal probe for performing colpotomies covered by the present invention consists of at least three distinct parts: a head at the distal end, a central stem and an ergonomic handle, which can accommodate a light source, being made in a single piece of a single transparent material to allow light to pass through.

The head is shaped like a cylinder at its end and like a truncated cone at its junction with the central stem, being hollow on the inside and having a dimension such that it must adapt to the size of the cervix, accommodating it completely inside without injuring it. On the inner side there are grooves of approximately semi-toroidal shape corresponding to external rings in relief on the outer side of the cylinder. These rings mark the exact length to be sectioned through the vagina.

The dimensions of the head can be adapted for different sizes of vagina, having as a non-limiting reference a cylinder height of 30 mm, a cone height of 30 mm, a cylinder inner diameter of 30 mm, an outer diameter of 35 mm and an outer ring relief of 1.5 mm, with three rings 10 mm apart, and the half-round grooves having a diameter of 1 mm.

The rings allow the vagina to be constricted by means of a knot-loop around it that completely occludes the vagina, preventing slippage at the moment of traction of the surgical specimen or hysterectomy, with the cervix being completely hidden inside the cylinder and occluded by the loop, and the cutting line must be placed below the loop (ring) to ensure the complete tightness of the cervix at the moment of extraction. Once the piece has been extracted, it would be necessary to cut the loop to separate the uterus-neck from the interior of the device. With this manoeuvre, the recently established oncological criteria on the importance of avoiding exposure of the tumour (cervical cancer) to the peritoneal cavity or the interior of the abdomen would be completely fulfilled.

Additionally, by illuminating the inside of the device with a light source, the rings produce a luminous glow through the vagina before this section and serve as a guide to the precise point of incision.

The central stem connects the head to the handle, being cylindrical in shape and hollow on the inside, with an inner diameter sufficient to allow the insertion of a video-laparoscopy optic (usually 10 mm in diameter) and an outer diameter such as to guarantee its mechanical strength and similar to the smaller diameter of the truncated cone. It is also long enough to be used as a lever shaft.

As a reference in order to meet the described needs, the stem can have a length between 180 and 200 mm, an outer diameter of about 11 mm and an outer diameter of about 16 mm.

Video-laparoscopy optics allow direct visualisation of the target to be encircled (cervix) on the screen, thus ensuring correct positioning of the device around the cervix, in the vaginal fornices, without any manipulation or trauma to the cervix.

In addition, the internal channel of the hollow stem allows the placement of grasping forceps (around 5-10 mm in diameter) to ensure distal neck traction in a joint extraction manoeuvre of the hysterectomy specimen and the manipulator. This allows the instruments to be used for other hysterectomy indications, such as benign pathologies and endometrial cancer, without the need to use the loop around the ring manoeuvre described in the case of requiring absolute tightness in the extraction of a tumour neck.

The handle has a cylindrical shape and a length that allows an ergonomic grip of the device that facilitates lateralisation and pulsation movements on the uterus through the vaginal fornices, completely avoiding invasion and manipulation of both the cervix and the interior of the uterine cavity. This makes it suitable for tumours of cervical origin as well as for tumours of endocavitary origin. The handle has a central hole of the same inner diameter as the central stem and is connected to it to allow the passage of the endoscope through it.

For reference, the handle can be around 40 mm in diameter by 100 mm in length with an inside diameter of 11 mm.

A medium warm LED light bulb (colour temperature of 4500K) can be housed in the handle which, taking advantage of the light-conducting properties of the transparent material, illuminates the walls of the device, with luminosity in the rings of the distal cylinder of the head which marks the vaginal transillumination incision area.

The material in which the probe is constructed is bio-compatible, resistant to temperatures of up to 120°C and with a resistance and flexibility suitable for the manoeuvres for which it has been designed. It is also semi-invariable from a socio-sanitary point of view, i.e. it can be washed and re-sterilised at least 15 to 20 times to ensure that it maintains its properties intact and at the same time is environmentally sustainable.

Thanks to all this, we have a probe that allows, in the case of cervical cancer, uterine manipulation without any trauma to the cervix, completely isolating the tumour surface inside the head by knotting the distal vagina with a loop on the ring of the device, immediately cranial to the cutting and sectioning area. In the case of cancer of the uterine corpus (endometrium, sarcomas, etc.), it avoids the introduction of the stem into the tumour cavity, which is oncologically adequate and completely reduces the possibility of uterine perforation and exposure of the tumour to the abdominal cavity. In the case of other benign pathologies that require hysterectomy (hypertrophic uterus, myomas, hyperplasia, etc.), in addition to the advantages already described, it ensures good pulsation of the vaginal fornices with the possibility of backlighting and marking the surface where the vaginal section must be performed.

Throughout the description and the claims, the word "comprises" and variants thereof are not intended to exclude other technical features, components, additives or steps. To those skilled in the art, other objects, advantages and features of the invention will be apparent in part from the invention and in part from the practice of the invention. The following examples and drawings are provided by way of illustration and are not intended to restrict the present invention. Furthermore, the invention covers all possible combinations of particular and preferred embodiments indicated herein.

### BRIEF DESCRIPTION OF THE DRAWINGS

To complement the description herein and to assist in a better understanding of the features of the invention, a set of drawings is attached hereto as an integral part of the said description, in which the following is illustratively and non-limitingly depicted:
Figure 1 shows an axonometric view of a preferred embodiment of the vaginal probe for performing colpotomies that is the subject of the present invention.
Figure 2 shows an elevation view of a preferred embodiment of the vaginal probe for performing colpotomies that is the subject of the present invention.
Figure 3 shows a longitudinal section view of a preferred embodiment of the vaginal probe for performing colpotomies that is the subject of the present invention.

### PREFERRED EMBODIMENT OF THE INVENTION

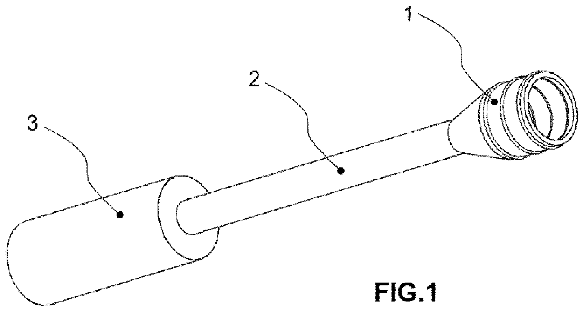

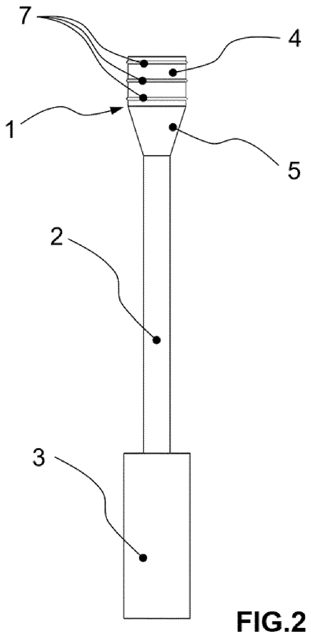

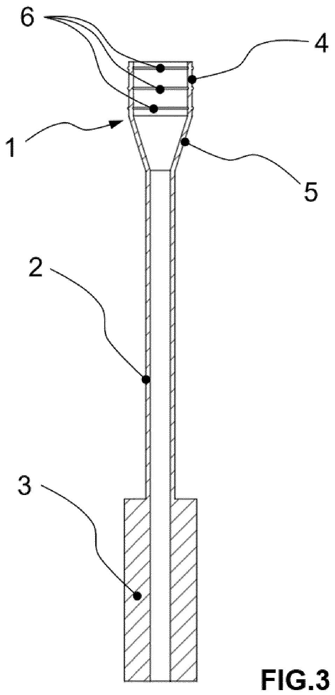

Using the numbering adopted in Figures 1, 2 and 3 of the present document to identify the elements comprising the vaginal probe for performing colpotomies covered by the present invention, we proceed to the description of these elements for a preferred embodiment of the invention.

In a preferred embodiment, the probe consists of at least three distinct parts: a head (1) at the distal end, a central stem (2) and an ergonomic handle (3), which can accommodate a light source, being made in a single piece of a single transparent material to allow light to pass through.

The head (1) is shaped like a cylinder (4) at its end and like a truncated cone (5) at its junction with the central stem (2), being hollow on the inside, having a dimension such that it must adapt to the size of the cervix, accommodating it completely inside without injuring it. On the inner side there are grooves (6) of semitoroidal shape in correspondence with some outer rings (7) in relief on the outer side of the cylinder (4). These rings mark the exact length to be sectioned through the vagina.

The dimensions of the head (1) can be adapted for different sizes of vagina, having in a preferred embodiment a height of the cylinder (4) of 30 mm, a height of the cone (5) of 30 mm, an inner diameter of the cylinder (4) of 30 mm and an outer diameter of 35 mm, and a relief of the outer rings (7) of 1,5 mm, three rings (7) being 10 mm apart and the half-round grooves (6) having a diameter of 1 mm.

The central stem (2) connects the head (1) with the handle (3), being cylindrical in shape and hollow on the inside, with an inner diameter sufficient to allow the introduction of a video-laparoscopy optic (usually 10 mm in diameter) and the placement of grasping forceps (around 5-10 mm in diameter) to ensure distal traction of the neck, as well as an outer diameter such as to guarantee its mechanical resistance and similar to the smaller diameter of the truncated cone (5). It also has a length such that it is used as a lever shaft, having in a preferred embodiment a length of between 180 and 200 mm, an inner diameter of 11 mm and an outer diameter of 16 mm.

The handle (3) has a cylindrical shape and a length that allows an ergonomic grip of the device that facilitates lateralisation and pulsation movements on the uterus through the vaginal fornices, completely avoiding invasion and manipulation of both the cervix and the interior of the uterine cavity. This makes it suitable for tumours of cervical origin as well as for tumours of endocavitary origin. The handle (3) has a central hole of the same inner diameter as the central hole and is connected to it to allow the passage of the endoscope through it, having in a preferred embodiment a diameter of 40 mm, 100 mm in length and an inner diameter of 11 mm.

In a preferred embodiment, the handle (3) houses a medium warm LED-type light bulb with a colour temperature of 4500K which, taking advantage of the light-conducting properties of the transparent material, illuminates the walls of the device, with particular luminosity in the area of the rings of the distal cylinder of the head that marks the vaginal transillumination incision area.

The material of which the probe is made is bio-compatible, resistant to temperatures of up to 120°C and with a resistance and flexibility suitable for the manoeuvres for which it has been designed, and which can be washed and re-sterilised at least 15-20 times to ensure that it maintains its properties intact and at the same time is environmentally sustainable, being, therefore, a semi-inventoriable material from the social and health point of view.

## Claims

1. Vaginal probe for performing colpotomies, **characterised in that** it consists of at least one head (1) at the distal end, a central stem (2) and an ergonomic handle (3), being made in one piece, the head (1) having the shape of a cylinder (4) at its end and a truncated cone (5) at its junction with the central stem (2), being hollow on the inside, the inner face of the head (1) has grooves (6) of approximately semitoroidal shape in correspondence with outer rings (7) in relief on the outer face of the cylinder (4), the central stem being a hollow cylinder with an outer diameter similar to the smaller diameter of the truncated cone (5) and the handle (3) having a larger diameter, the handle (5) also being hollow with an inner diameter similar to the inner diameter of the central stem (2), its dimensions being able to adapt to the different sizes of vagina.

2. Vaginal probe for performing colpotomies according to claim 1 **wherein** it is made of a single transparent material to allow light to pass through.

3. Vaginal probe for performing colpotomies according to claim 2, **where** the handle (3) houses an LED-type light bulb which, taking advantage of the light-conducting properties of the transparent material, illuminates the walls of the probe, with particular luminosity in the area of the rings of the cylinder (4) distal to the head (1).

4. Vaginal probe for performing colpotomies according to claim 3 **wherein** the LED type lamp has a medium warmth with a colour temperature value of 4500 K.

5. Vaginal probe for performing colpotomies according to claim 1 **wherein** it is made of a single bio-compatible material, resistant to temperatures up to 120°C, as well as washable and sterilisable at least 15 to 20 times.

6. Vaginal probe for performing colpotomies according to claim 1 **wherein** the cylinder (4) of the head (1) has a height of 30 mm, a height of the cone (5) of 30 mm and an inner diameter of the cylinder (4) of 30 mm and an outer diameter of 35 mm, with a relief of the outer rings (7) of 1.5 mm, having three rings (7) spaced 10 mm apart.

7. Vaginal probe for performing colpotomies according to claim 1 **wherein** the central stem (2) has a length between 180 and 200 mm, an inner diameter of 11 mm and an outer diameter of 16 mm.

8. Vaginal probe for performing colpotomies according to claim 1 wherein the handle has an outer diameter of 40 mm, a length of 100 mm and an inner diameter of 11 mm.
